# EUROPEAN PATENT APPLICATION

(11) **EP 4 574 029 A1**
(43) Date of publication of application: **25.06.2025**
(21) Application number: 23872818.2
(22) Date of filing: 28.08.2023
(51) Int. Cl.: A61B 5/024, A61B 5/1455, A61B 5/00

(54) **RING-SHAPED ELECTRONIC DEVICE COMPRISING OPTICAL COATING LAYER**

(30) Priority: 26.09.2022 KR 20220122008; 10.11.2022 KR 20220149980
(71) Applicant: Samsung Electronics Co., Ltd., Suwon-si, Gyeonggi-do 16677 (KR); Research & Business Foundation Sungkyunkwan University, Suwon-si, Gyeonggi-do 16419 (KR)
(72) Inventor: BAAC, Hyoungwon, Suwon-si Gyeonggi-do 16419 (KR); LIM, Daehyeong, Suwon-si Gyeonggi-do 16677 (KR); JOO, Mingyu, Suwon-si Gyeonggi-do 16419 (KR)
(74) Representative: Gulde & Partner
(86) International application number: PCT/KR2023/012727
(87) International publication number: WO 2024/071696

(57) **Abstract**

This electronic device may comprise: a ring-shaped housing having an inner surface onto which a user's finger is inserted; a light-emitting unit configured to emit light from the inside of the housing to the inner surface of the housing through a first region of the inner surface of the housing; a light-receiving unit configured to receive first light, which is a part of the light reflected from the inside of the user's finger, within the housing through a second region of the inner surface of the housing; and a coating layer disposed between the first region and the second region and configured to reflect second light, which is separated from the first light and is the other part of the light, to the user's finger so as to transfer same to the light-receiving unit. Other embodiments are also possible.

## Description

### [Technical Field]

The present disclosure relates to a ring-shaped electronic device comprising an optical coating layer.

### [Background Art]

An electronic device may include sensors for obtaining information around the electronic device to provide various functions to a user. A wearable electronic device worn by the user may include sensors for obtaining a biometric information of a user. For example, the electronic device may include an optical sensor to obtain the biometric information of the user.

### [Disclosure]

### [Technical Solution]

According to an embodiment, an electronic device may comprise a ring-shaped housing. The housing may have an inner surface into which a finger of a user is inserted. The electronic device may include a light-emitting unit. The light-emitting unit may be configured to emit light toward the inner surface of the housing through a first region of the inner surface of the housing within the housing. The electronic device may include a light-receiving unit. The light-receiving unit may be configured to receive a first light, which is a portion of the light reflected from the inside of the finger of the user, through a second region of the inner surface of the housing within the housing. The electronic device may include a coating layer. The coating layer may be disposed between the first region and the second region, and may be configured to reflect a second light, which is distinct from the first light and is remaining portion of the light, to the finger of the user to transmit the second light to the light-receiving unit.

According to an embodiment, the electronic device may include a housing. The housing may be formed in a ring-shaped having an inner surface into which a finger of a user is inserted. The electronic device may include a cover. The cover may form at least a portion of the inner surface of the housing, and may include a transparent portion. The electronic device may include a light-emitting unit. The light-emitting unit may be configured to emit light toward the cover through a first region of the cover within the housing. The electronic device may include a light-receiving unit. The light-receiving unit may be configured to receive a first light reflected from the inside of the finger of the user through a second region of the cover within the housing. The electronic device may include an additional light-receiving unit. The additional light-receiving unit may be disposed between the light-receiving unit and the light-emitting unit, and may be configured to receive a second light, which is distinct from the first light and is reflected from an inside of the finger of the user, through a third region of the cover within the housing. The electronic device may include a coating layer. The coating layer may be disposed on the cover and between the first region and the second region, and may be configured to reflect a third light, which is distinct from the first light and the second light and is reflected from an inside of the finger of the user, to the finger of the user to transmit the third light to the light-receiving unit. The electronic device may include an additional coating layer. The additional coating layer may be different from the coating layer and may be disposed between the coating layer and the first region.

### [Description of the Drawings]

FIG. 1 is a perspective view indicating an exemplary state in which an electronic device is worn on a portion of a body of a user according to an embodiment.
FIG. 2 is a schematic diagram of an exemplary electronic device according to an embodiment.
FIG. 3 is a schematic diagram of an exemplary electronic device in which another light-emitting unit different from a light-emitting unit is added according to an embodiment.
FIG. 4 is a schematic diagram of an exemplary electronic device to which another light-receiving unit distinct from the light-receiving unit is added according to an embodiment.
FIG. 5 is a diagram illustrating a path through which a light emitted from a light-emitting unit is transmitted to a light-receiving unit according to an embodiment.
FIG. 6 is a block diagram of an electronic device within a network environment according to one embodiment.

### [Mode for Invention]

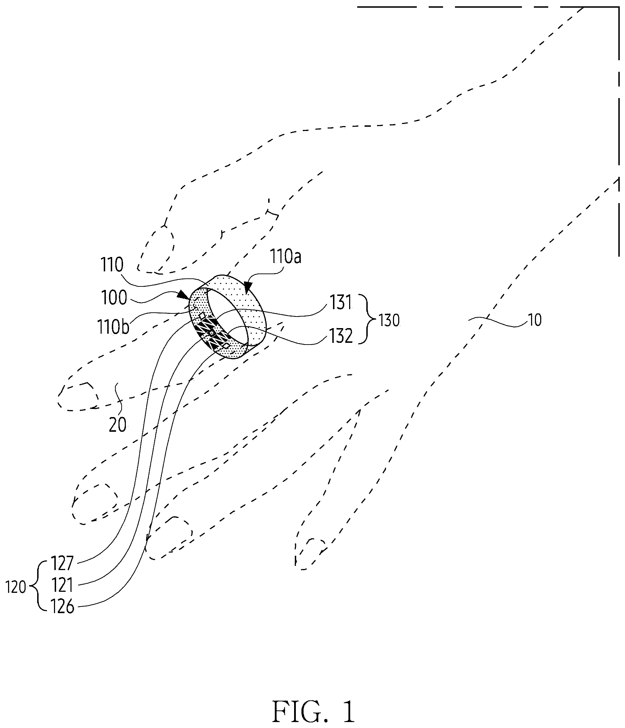

FIG. 1 is a perspective view indicating an exemplary state in which an electronic device is worn on a portion of a body of a user according to an embodiment.

Referring to FIG. 1, an electronic device 100 may be worn on the portion of the body of the user. The electronic device 100 may be referred to as a wearable device in terms of being worn on the portion of the body of the user. The electronic device 100, which is the wearable device, may obtain biometric information of the user. The electronic device 100 may transmit the obtained biometric information to another electronic device. The other electronic device may transmit to a portable terminal (e.g., a smartphone or a tablet PC) of the user, or may include a PC. The other electronic device may be an electronic device designated by a user. The biometric information transmitted to the other electronic device may be secured. The biometric information may include a heart rate, heart rate defibrillation, a body fat percentage, an oxidation saturation, or a blood alcohol concentration of the user.

According to an embodiment, the electronic device 100 may include a housing 110 forming an outer surface of the electronic device 100. The housing 110 may be formed to be wearable on a portion (e.g., a finger) of a body of a user 10. The electronic device 100 may include a cover forming at least a portion of an inner surface of the housing 110 and including a transparent portion. The transparent portion may penetrate a light. For example, the electronic device 100 may be worn on one of fingers 20 of the user 10. The housing 110 may have a ring shape to be worn on the finger 20 of the user 10. The housing 110 may include an outer surface 110a forming an outer side of the ring shape and an inner surface 110b forming an inner side of a ring shape. The outer surface 110a may be an outer circumferential surface of the housing 110. A portion of the outer surface 110a of the housing 110 may be formed of a material that the inside is not visible. A portion of the outer surface 110a of the housing 110 may include a display. For example, a region of the outer surface 110a in which the display is disposed may provide visual information. The region of the outer surface 110a in which the display is disposed may include a transparent material. For example, the electronic device 100 may may include the cover forming at least a portion of the inner surface of the housing 110 and including the transparent portion. The transparent portion may penetrate a light.

According to an embodiment, the inner surface 110b may be an inner circumferential surface of the housing 110. The inner surface 110b may be a surface that the finger 20 of the user 10 contacts while the user 10 wears the electronic device 100. The inner surface 110b may include a transparent region for an operation of an optical sensor 120.

According to an embodiment, the electronic device 100 may include the optical sensor 120 and a coating layer 130. The optical sensor 120 may be disposed inside the housing 110. The optical sensor 120 may be disposed between the outer surface 110a and the inner surface 110b to face the inner surface 110b. The housing 110 may include a transparent portion disposed in a region of the inner surface 110b in which a light-emitting unit 121 and light-receiving units 126 and 127 of the optical sensor 120 are disposed. The optical sensor 120 may be a photoplethysmogram (PPG) sensor configured to obtain the biometric information from the body of the user. The PPG sensor may be configured to emit a light to the body of the user and receive a light reflected from the body of the user to detect a change in a blood flow in a microvasculature. The PPG sensor may be configured to emit the light to the body of the user and receive the light reflected from the body of the user to detect the change in the blood flow in the microvasculature. For example, as an amount of a blood flow inside microvasculature of the user changes by periodic contraction or relaxation of a heart, a volume of the microvasculature of the body of the user may change. An amount of the light emitted from the PPG sensor and penetrated to the body of the user that is absorbed by blood vessels of the user may be changed according to a change in an amount of a blood flow of a portion of the body of the user. The PPG sensor may be configured to obtain biometric data related to the body of the user based on identifying an intensity of the light reflected from the body of the user. For example, the biometric data may include at least one of a change in the heart rate, the oxidation saturation, and a blood pressure of the user during designated time. According to an embodiment, the optical sensor 120 may include the at least one light-emitting unit 121 and the light-receiving units 126 and 127 configured to obtain the biometric information from the body of the user. The light-emitting unit 121 may include a laser, a light emitting diode (LED), and an organic light emitting diode (OLED). For example, the light emitted from the light-emitting unit 121 may reach into a tissue of the body, by penetrating a portion of the finger 20 of the user 10. For example, the light may reach a designated material (e.g., glucose in a blood or an alcohol molecule in the blood) in the body. After a wavelength and/or an intensity of the light reaching the designated material is changed, the light emitted from the light-emitting unit 121 is reflected from the tissue inside the finger 20 of the user 10, and the reflected light may be transmitted to the light-receiving units 126 and 127. The optical sensor 120 may obtain another biometric data related to the body of the user based on receiving the light reflected from the portion (e.g., blood vessels) of the body of the user. For example, another biometric data may mean the heart rate, the oxidation saturation, a blood sugar level, or the blood alcohol concentration in the body of the user.

According to an embodiment, as the light emitted from the light-emitting unit 121 is reflected inside the finger 20 and transmitted to the light-receiving units 126 and 127, the optical sensor 120 may obtain the biometric data. In order to increase accuracy of the biometric data, the electronic device 100 may include the coating layer 130 to reduce a loss of the light transmitted to the light-receiving units 126 and 127. The coating layer 130 may transmit a portion of the light transmitted to a region other than the light-receiving units 126 and 127 among lights emitted from the light-emitting unit 121 and reflected from the inside of the finger 20 back to the inside of the finger 20. The coating layer 130 may be a coating that reflects a light. For example, the coating layer 130 may reflect a light reaching the coating layer 130 from the inside of the finger 20 back to the inside of the finger 20. The light reflected back to the inside of the finger 20 may be reflected from the inside of the finger 20 and transmitted to one of the light-receiving units 126 and 127. As a proportion of the lights transmitted to the light-receiving units 126 and 127 among the lights emitted from the light-emitting unit 121 increases, the light-receiving units 126 and 127 may increase accuracy of a biometric signal.

According to an embodiment, the coating layer 130 may be disposed between the light-emitting unit 121 and the light-receiving units 126 and 127. For example, the first coating layer 131 may be disposed between the light-emitting unit 121 and the first light-receiving unit 126. The second coating layer 132 may be disposed between the light-emitting unit 121 and the second light-receiving unit 127. The first coating layer 131 may reflect a portion of a light that is reflected and does not reach the first light-receiving unit 126 among the lights emitted from the light-emitting unit 121 back to the inside of the finger 20. The second coating layer 132 may reflect a portion of a light that is reflected and does not reach the first light-receiving unit 126 among the lights emitted from the light-emitting unit 121 back to the inside of the finger 20.

The electronic device 100, according to the above-described embodiment, may obtain data related to the biometric signal of the user through the optical sensor 120. The electronic device 100 may increase an amount of the light transmitted to the light-receiving units 126 and 127 by reflecting the light that may not be transmitted to the light-receiving units 126 and 127 back to the inside of the body of the user 10. As the amount of the light transmitted to the light-receiving units 126 and 127 increases, the electronic device 100 may increase accuracy of the data related to the biometric signal obtained through the optical sensor 120.

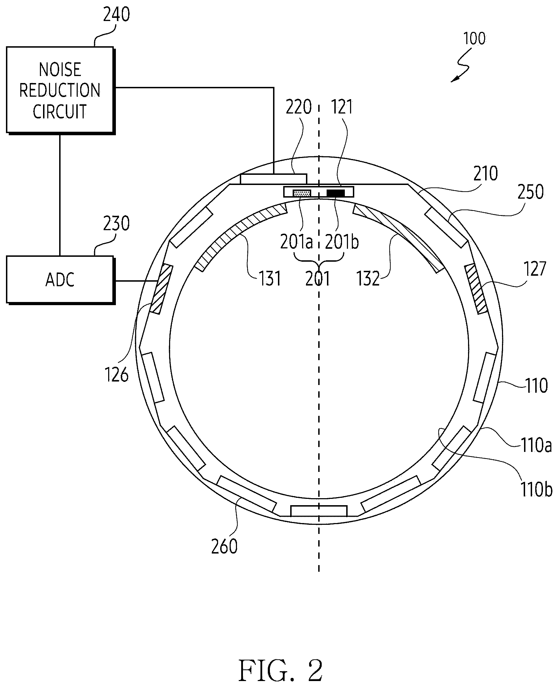

FIG. 2 is a schematic diagram of an exemplary electronic device according to an embodiment.

Referring to FIG. 2, an electronic device 100 may include a housing with an inner surface 110b into which a finger (e.g., the finger 20 of FIG. 1) of a user (e.g., the user 10 of FIG. 1) is inserted, a light-emitting unit 121 configured to emit a light to the finger 20 of the user 10, light-receiving units 126 and 127 configured to receive a portion of a light reflected from the inside of the finger 20 of the user 10, and coating layers 131 and 132, which are disposed between a partial region of the inner surface 110b through which a light emitted from the light-emitting unit 121 passes and a another partial region of the inner surface 110b transmitted to the light-receiving units 126 and 127.

According to an embodiment, a housing 110 may form an outer surface 110a of the electronic device 100 and may be formed in a hollow ring shape. Electronic components may be disposed in a space between the outer surface 110a and the inner surface 110b of the housing 110. For example, the electronic components disposed in the housing 110 may include an optical sensor (e.g., the optical sensor 120 of FIG. 1), a processor 220, an analogue digital converter (ADC) 230, a noise reduction circuit 240, a wireless communication circuit 250, and a battery 260.

According to an embodiment, the light-emitting unit 121 and the light-receiving units 126 and 127 may obtain data related to a biometric signal of the user. For example, as the light emitted from the light-emitting unit 121 is reflected from the inside of the finger 20 and reaches the light-receiving units 126 and 127, the processor 220 may obtain data related to the biometric signal through the light-receiving units 126 and 127. Each of the light-receiving units 126 and 127 may be spaced apart from the light-emitting unit 121. An angle between the light-receiving units 126 and 127 and the light-emitting unit 121 with respect to a center of a ring shape may be 50 degrees to 70 degrees. The angle between the light-emitting unit 121 and the first light-receiving unit 126 with respect to a center of a circle formed by the inner surface 110b may be approximately 60 degrees. The angle between the light-emitting unit 121 and the second light-receiving unit 127 with respect to the center of the circle formed by the inner surface 110b may be approximately 60 degrees.

According to an embodiment, the light-emitting unit 121 may contact a lower surface of the finger 20 when the user wears the electronic device 100. The lower surface of the finger 20 may be the same as a direction in which a palm faces. The light-receiving units 126 and 127 may include the optical sensor. The light-receiving units 126 and 127 may convert the light reaching the light-receiving units 126 and 127 into a current.

According to an embodiment, the light-emitting unit 121 and the light-receiving units 126 and 127 may be disposed on a flexible printed circuit board (FPCB) 210. The FPCB 210 may be disposed inside the housing 110. The FPCB 210 may be bent to correspond to a shape of the housing 110. The light-emitting unit 121, the light-receiving units 126 and 127, the processor 220, the ADC 230, the noise reduction circuit 240, the wireless communication circuit 250, and the battery 260 may be disposed in the FPCB 210. The processor 220 may include one or more of a central processing unit, an application processor, a graphic processing unit (GPU), an application processor, a sensor processor, and a communication processor.

According to an embodiment, the ADC 230 may convert an analog signal into a digital signal. The ADC 230 may be electrically connected to the light-receiving units 126 and 127. A signal obtained by the light-receiving units 126 and 127 may be the analog signal. The ADC 230 may convert sensing data of the light-receiving units 126 and 127 into digital data. The converted data signal may be transmitted to the processor 220 through the noise reduction circuit 240.

According to an embodiment, the noise reduction circuit 240 may be configured to remove noise from among the sensing data detected by the light-receiving units 126 and 127. For example, the light-receiving units 126 and 127 may detect a light transmitted along a surface of the inner surface 110b of the housing 110 or a light introduced from the outside of the electronic device 100, in addition to a light reflected by a tissue (e.g., a bone or blood vessel) inside the finger 20 of the user 10. The light introduced from the outside or the light transmitted along the surface of the inner surface 110b may interfere with accurate detection of the biometric signal. The noise reduction circuit 240 may include a filter circuit or a PID control circuit. The filter circuit may extract a light corresponding to the noise. For example, a light having a lower frequency than the light emitted from the light-emitting unit 121 may be removed. For example, the light-emitting unit 121 may be a high pass filter. In order to remove a current generated by an external light, the PID control circuit may generate a reverse current value and provide a current based on the generated reverse current value. The current generated by the external light may be offset by the provided current.

According to an embodiment, the wireless communication circuit 250 may supply a signal or power to an antenna (not illustrated) within the electronic device 100. The antenna may include a near field communication (NFC) antenna, a wireless charging antenna, and a magnetic secure transmission (MST) antenna. The antenna may perform short-range communication with an external device, wirelessly transmit and receive power required for charging, and transmit a magnetic-based signal including a short-range communication signal or payment data. The wireless communication circuit 250 may transmit a signal to an antenna radiator formed by a portion of the housing 110. The wireless communication circuit 250 may wirelessly communicate with another electronic device of the user through the antenna or the antenna radiator.

According to an embodiment, the battery 260 is a device for supplying power to at least one component of the electronic device 100, and may include, for example, a non-rechargeable primary battery, a rechargeable secondary battery, or a fuel cell. At least a portion of the battery 260, for example, may be disposed on substantially the same plane as the FPCB 210. The battery 260 may be integrally disposed inside the electronic device 100 or may be disposed to be detachably from the electronic device 100.

According to an embodiment, the electronic device 100 may further include memory and/or an interface mounted on the FPCB 210. The memory may include, for example, volatile memory or non-volatile memory. The interface may include, for example, a high definition multimedia interface (HDMI), a universal serial bus (USB) interface, a SD card interface, and/or an audio interface. The interface, for example, may electrically or physically connect the electronic device 100 to an external electronic device, and may include a USB connector, an SD card/MMC connector, or an audio connector.

According to an embodiment, the light-emitting unit 1210 may include a plurality of light sources 201. A first light source 210a may emit a light of a first wavelength band, and a second light source 210b may emit a light of a second wavelength band different from the light of the first wavelength band. For example, the first light source 210a may emit a red light, and the second light source 210b may emit an infrared light. The red light and the infrared light may be used to measure an oxidation saturation or a blood alcohol concentration.

According to an embodiment, the coating layers 131 and 132 may be disposed in a portion of the inner surface 110a of the housing 110. The coating layers 131 and 132 may be disposed between a region of the inner surface 110a overlapping the light-emitting unit 121 and a region of the inner surface 110b overlapping the light-receiving units 126 and 127. For example, the first coating layer 131 may be disposed between the region of the inner surface 110b overlapping the light-emitting unit 121 and a region of the inner surface 110a overlapping a first light-receiving unit 126. The second coating layer 132 may be disposed between the region of the inner surface 110b overlapping the light-emitting unit 121. The coating layers 131 and 132 may be a reflective coating. The coating layers 131 and 132 may reflect a light reaching the coating layers 131 and 132 among external light or a light reflected from the inside of the finger 20 of the user 10.

According to an embodiment, the light emitted from the light-emitting unit 121 and reflected from the inside of the finger 20 to reach the coating layers 131 and 132 may be reflected back to the inside of the finger 20. The light reflected by the coating layers 131 and 1320 may be reflected again by the tissue inside the finger 20 and transmitted to the light-receiving units 126 and 127.

According to an embodiment, the processor 220 may request the light-emitting unit 121 to emit lights to the outside of the electronic device 100 through the light source 201. For example, the processor 220 may transmit a signal requesting obtaining of the biometric signal to the optical sensor (e.g., the optical sensor 120 of FIG. 1) including the light-emitting unit 121 based on an event. The event may include an input related to a biometric signal measurement request inputted to the electronic device 100, a reception of an input through an external electronic device communicating with the electronic device 100, or an input provided based on a pre-designated period.

According to an embodiment, the processor 220 may receive a light emitted from light sources 201a and 201b of the light-emitting unit 121 through the light-receiving units 126 and 127. Through the light-receiving units 126 and 127, the processor 220 may obtain the biometric signal of the user 10 based on the received light. The processor 220 may obtain a signal from which data related to an external light is removed through the noise reduction circuit 240 based on the signal converted by the ADC 230. The processor 220 may obtain the biometric signal of the user based on the signal from which noise has been removed.

The electronic device 100, according to the above-described embodiment, may obtain the biometric signal of the user through the electronic device 100 including the light-emitting unit 121 and the light-receiving units 126 and 127. The electronic device 100 may guide a light that is not transmitted to the light-receiving units 126 and 127 to be reflected back to the finger 20 of the user 10 and transmitted to the light-receiving units 126 and 127 by including the coating layers 131 and 132. The electronic device 100 may improve a performance of the optical sensor 120 through the coating layers 131 and 132.

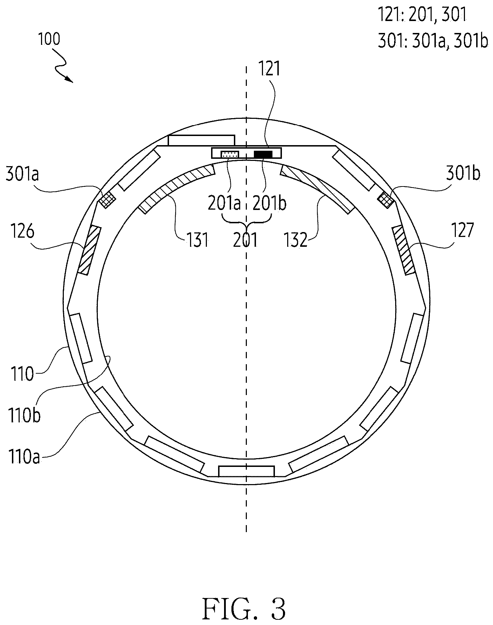

FIG. 3 is a schematic diagram of an exemplary electronic device in which another light-emitting unit different from a light-emitting unit is added according to an embodiment.

Referring to FIG. 3, an electronic device 100 may include a housing 110 forming at least a portion of an outer surface 110a and an inner surface 110b of the electronic device 100, a light-emitting unit 121, light-receiving units 126 and 127, and coating layers 131 and 132. The light-emitting unit 121 and the light-receiving units 126 and 127 may be disposed to face the inner surface 110b of the housing 110. The coating layers 131 and 132 may be disposed in the inner surface 110b of the housing 110.

According to an embodiment, the light-emitting unit 121 of the electronic device 100 may include a plurality of light sources. The light-emitting unit 121 may include an additional light-emitting unit 301. The additional light-emitting unit 301 may include a light source different from a light source of the light-emitting unit 121. For example, the light-emitting unit 121 may include a first light source 201. The first light source 201 may emit a light in a first wavelength band. The first light source 201 may include a plurality of light sources. For example, the first light source 201 may include a red light source 201a that emits a light of a red wavelength and an infrared light source 201b that emits a light of an infrared or a near infrared wavelength. The additional light-emitting unit 301 may include second light sources 301a and 301b. The second light sources 301a and 301b may emit a light having a shorter wavelength than the first light source 201. For example, the second light sources 301a and 301b may emit light having a green wavelength. The second light sources 301a and 301b may be formed as a module separate from the light-emitting unit 121. The second light sources 301a and 301b formed as the module separate from the light-emitting unit 121 may be referred to as an additional light-emitting unit or an auxiliary light-emitting unit in terms of operating as the separate light-emitting unit 121.

According to an embodiment, the second light sources 301a and 301b may be spaced apart from the light-emitting unit 121 and disposed in both sides of the light-emitting unit 121. For example, the one 301a of the second light sources 301a and 301b may be spaced apart from the light-emitting unit 121 to left on the drawing, and the other one 301b of the second light sources 301a and 301b may be spaced apart from the light-emitting unit 121 to right on the drawing. The light-receiving units 126 and 127 may receive a light reflected from the inside of the body of the user among lights emitted from the first light source 201 and the second light sources 301a and 301b of the light-emitting unit 121. For example, the light-receiving units 126 and 127 may be configured to receive a portion of a red light or an infrared light emitted from the first light source 201 and reflected from the inside of a finger and may be configured to receive a portion of a green light emitted from the second light sources 301a and 301b and reflected from the inside of the finger.

According to an embodiment, the light-receiving units 126 and 127 may be configured to obtain data related to an oxidation saturation of the user using the infrared light or the red light, and obtain data related to a heart rate of the user using the green light. For example, the green light may have a shorter wavelength than the red light or the infrared light. A light having a short wavelength may have a shorter penetration depth inside the body compared to a light having a longer wavelength. The green light may be used to measure the heart rate. The red light or the infrared light may be used to measure the oxidation saturation or a glucose in blood vessels. The green light may be reflected between epidermis and dermis. The red light or the infrared light may be reflected from a tissue inside a skin by penetrating the dermis. The green light may have a short progression distance inside the finger of the user 10, and the reflected green light may be reflected at a close distance from the second light sources 301a and 301b. The red light or the infrared light may have a long progression distance inside the finger of the user 10, and the reflected red light or infrared light may be reflected by a far distance from the first light source 201. The second light sources 301a and 301b may be disposed adjacent to the light-receiving units 126 and 127 to increase a reception rate of the green light. The first light source 201 may be disposed relatively far from the light-receiving units 126 and 127 to increase a reception rate of the red light or the infrared light. According to an embodiment, the second light sources 301a and 301b may be disposed between the light-receiving units 126 and 127 and the light-emitting unit 121. For example, the one light source 301a of the second light sources 301a and 301b may be disposed between the first light-receiving unit 126 and the light-emitting unit 121, and the other light source 301b may be disposed between the second light-receiving unit 127 and the light-emitting unit 121.

According to an embodiment, the electronic device 100 may include the coating layers 131 and 132 to increase a light transmitted to the light-receiving units 126 and 127. The coating layers 131 and 132 may be disposed between the second light sources 301a and 301b and the light-emitting unit 121. For example, the first coating layer 131 may be disposed between a region of the inner surface 110b overlapping the light-emitting unit 121 and a region of the inner surface 110b overlapping the one light source 301a among the second light sources 301a and 301b. A second coating layer 132 may be disposed between the region of the inner surface 110b overlapping the light-emitting unit 121 and a region of the inner surface 110b overlapping the other light source 301b among the second light sources 301a and 301b.

According to the above-described embodiment, the electronic device 100 may increase accuracy of biometric data obtained through the light-receiving units 126 and 127 by including the coating layers 131 and 132. The electronic device 100 may increase the accuracy of the data related to the heart rate obtained through the light-receiving units 126 and 127 by disposing the second light sources 301a and 301b emitting the green light adjacent to the light-receiving units 126 and 127.

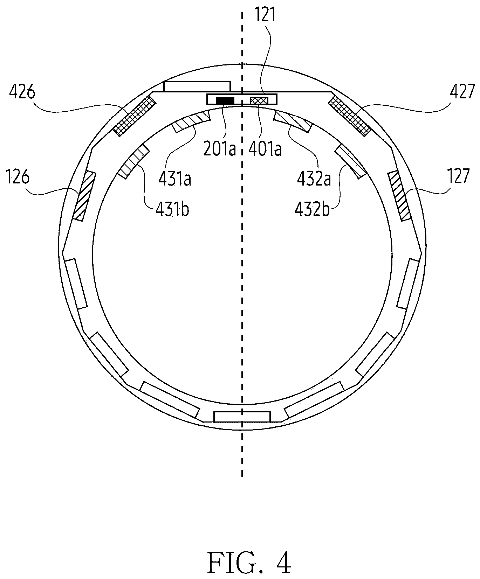

FIG. 4 is a schematic diagram of an exemplary electronic device to which another light-receiving unit distinct from the light-receiving unit is added according to an embodiment.

Referring to FIG. 4, an electronic device 100 may include a housing 110 forming at least a portion of an outer surface 110a and an inner surface 110b of the electronic device 100, a light-emitting unit 121, light-receiving units 126 and 127, and coating layers 431a, 431b, 432a and 432b. The light-emitting unit 121 and the light-receiving units 126 and 127 may be disposed to face the inner surface 110b of the housing 110. The coating layers 131 and 132 may be disposed in the inner surface 110b of the housing 110.

According to an embodiment, the light-emitting unit 121 of the electronic device 100 may include a plurality of light sources. For example, the light-emitting unit 121 may include a red light source 201a that emits at least one of a red light or an infrared light and a green light source 401a that emits a green light. The red light source 201a includes a light source that emits a red light but may be a light source that emits an infrared light. The light-emitting unit 121 may further include an infrared light source distinct from the red light source 201a. The light-emitting unit 121 may dispose a plurality of light sources in one module.

According to an embodiment, the electronic device 100 may further include the light-receiving units 126 and 127 and additional light-receiving units 426 and 427 different from the light-receiving units 126 and 127. The light-receiving units 126 and 127 may be configured to receive a portion of a first wavelength of a light emitted from the red light source 201a reflected from the inside of the finger. The additional light-receiving units 426 and 427 may be configured to receive a portion of a second wavelength of a light emitted from the green light source 401a and reflected from the inside of the finger. The light-receiving units 126 and 127 may measure an oxidation saturation, a blood sugar, or a blood alcohol concentration of a user, by using the light emitted from the red light source 201a emitting the red light or the infrared light. The additional light-receiving units 426 and 427 may measure a heart rate of the user by using the light emitted from the green light source 401a that emits the green light. The green light may be reflected between epidermis and dermis. The red light or the infrared light may penetrate the dermis and be reflected from a tissue inside a skin. The green light may have a short progression distance inside the finger of the user 10, and the reflected green light may be reflected at a close distance the green light sources 401a. The red light or the infrared light may have a long progression distance inside the finger of the user 10, and the reflected red light or infrared light may be reflected by a far distance from the red light source 201a.

According to an embodiment, the additional light-receiving units 426 and 427 may include an optical sensor capable of sensing the green light. The optical sensor may include a photodiode. The additional light-receiving units 426 and 427 may be disposed adjacent to the light-emitting unit 121 to increase the reception rate of the green light. For example, the additional light-receiving units 426 and 427 may be disposed between the light-receiving units 126 and 127 and the light-emitting unit 121. For example, the first additional light-receiving unit 426 may be disposed between the first light-receiving unit 126 and the light-emitting unit 121, and the second additional light-receiving unit 427 may be disposed between the second light-receiving unit 127 and the light-emitting unit 121.

According to an embodiment, the electronic device 100 may include the coating layers 431a, 431b, 432a, and 432b to increase a light transmitted to the light-receiving units 126, 127, 426, and 427. The coating layers 431a, 431b, 432a and 432b may be disposed between the light-receiving units 126, 127, 426, and 427. For example, the first coating layer 431a may be disposed between a region of the inner surface 110b overlapping the light-emitting unit 121 and a region of the inner surface 110b overlapping the first additional light-receiving unit 426. The second coating layer 431b may be disposed between a region of the inner surface 110b overlapping the first additional light-receiving unit 426, and the region of the inner surface 110b overlapping the first light-receiving unit 126. The third coating layer 432a may be disposed between the region of the inner surface 110b overlapping the light-emitting unit 121 and a region of the inner surface 110b overlapping the second additional light-receiving unit 427. The fourth coating layer 432b may be disposed between the region of the inner surface 110b overlapping the second additional light-receiving unit 427, and the region of the inner surface 110b overlapping the second light-receiving unit 127.

According to an embodiment, the first coating layer 431a and the third coating layer 432a may reflect the green light emitted from the green light source 401a and transmitted to the coating layers 431a and 432a and then may be reflected to the inside of the finger of the user. The reflected green light may be reflected from the inside of the finger of the user and transmitted to the first additional light-receiving unit 426 or the second additional light-receiving unit 427.

According to an embodiment, not only the green light but also the red light may reach the first coating layer 431a and the second coating layer 432a. The first coating layer 431a and the third coating layer 432a may reflect the red light or the infrared light reaching the first coating layer 431a and the third coating layer 432a. The first coating layer 431a and the third coating layer 432a may reflect the red light emitted from the red light source 201a and transmitted to the coating layers 431a and 432a and then may be reflected to the inside of the finger of the user. The reflected red light may be reflected from the inside of the finger of the user and transmitted to the first light-receiving unit 126 or the second light-receiving unit 127.

According to an embodiment, the second coating layer 431b and the fourth coating layer 432b may reflect the red light or the infrared light reaching the second coating layer 431b and the fourth coating layer 432b. The second coating layer 431b and the fourth coating layer 432b may reflect the red light emitted from the red light source 201a and transmitted to the coating layers 431b and 432b and then may be reflected to the inside of the finger of the user. The reflected red light may be reflected from the inside of the finger of the user and transmitted to the first light-receiving unit 126 or the second light-receiving unit 127.

According to the above-described embodiment, the electronic device 100 may increase accuracy of biometric data obtained through the light receiving units 126 and 127 by including the coating layers 431a, 431b, 432a, and 432b.. The electronic device 100 may increase the accuracy of the data related to the heart rate obtained through the additional light-receiving units 426 and 427 by disposing the additional light-receiving units 426 and 427 adjacent to the light-emitting unit 121.

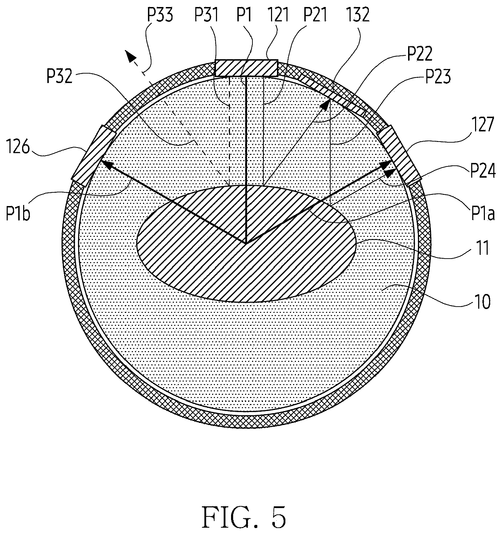

FIG. 5 is a diagram illustrating a path through which a light emitted from a light-emitting unit is transmitted to a light-receiving unit according to an embodiment.

Referring to FIG. 5, a portion of lights P1, P21, and P31 emitted from a light-emitting unit 121 may be reflected from a tissue 11 inside a body of a user 10 and transmitted to light-receiving units 126 and 127.

According to an embodiment, a portion of the lights P1, P21, and P31 emitted from the light-emitting unit 121 may be reflected from the inside of the tissue 11 and reflected to a designated position (e.g., the light-receiving units 126 and 127). For example, the light P1 may be reflected to the first light-receiving unit 126 along a path P1b or reflected to the second light-receiving unit 127 along a path P1a, by being reflected from the inside of the tissue 11. The light P1 transmitted to the first light-receiving unit 126 and the second light-receiving unit 127 may provide data related to a biometric signal to the first light-receiving unit 126 or the second light-receiving unit 127. The data related to the biometric signal may include an intensity, a wavelength change, or a magnitude change of the light P1. A portion of the lights P21 and P31 may be reflected from the tissue 11 and may not be transmitted to the light-receiving units 126 and 127. For example, a portion of the light P21 may be reflected from the tissue 11 and transmitted to a second coating layer 132. The second coating layer 1320 may reflect a reflected light P22 back to the inside of the body of the user 10. A light P23 reflected to the inside of the body may be reflected by the tissue 11 and transmitted to the second light-receiving unit 127. A remaining portion P31 of the light indicates a case reflected to a region without a coating layer. The remaining portion P31 of the light may be reflected from the tissue 11 and reflected to an inner surface of an electronic device 100. In case that there is no coating layer in the inner surface of the electronic device 100, a light may be reflected to the outside of the electronic device 100. For example, a light P32 reflected from the tissue 11 may reach the inner surface of the electronic device 100 without the coating layer, and the light P32 reaching the inner surface of the electronic device 100 may penetrating and may be transmitted to the outside of the electronic device 100. The electronic device 100 may include coating layers 131 and 132 between the light-receiving units 126 and 127 and the light-emitting unit 121 to reduce a light P33 transmitted to the outside. The electronic device 100 including the first coating layer 131 disposed between the first light-receiving unit 126 and the light-emitting unit 121 may reflect the light P32 back to the inside of the body of the user 10.

According to the above-described embodiment, the electronic device 100 may increase accuracy of biometric data obtained through the light-receiving units 126 and 127 by including the coating layers 131 and 132.

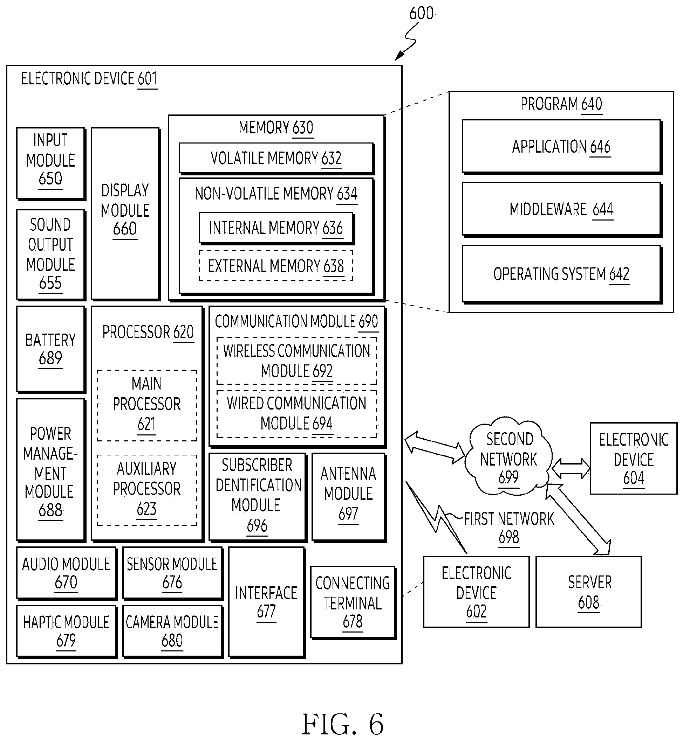

Fig. 6 is a block diagram illustrating an electronic device 601 in a network environment 600 according to various embodiments.

Referring to Fig. 6, the electronic device 601 in the network environment 600 may communicate with an electronic device 602 via a first network 698 (e.g., a short-range wireless communication network), or an electronic device 604 or a server 608 via a second network 699 (e.g., a long-range wireless communication network). According to an embodiment, the electronic device 601 may communicate with the electronic device 604 via the server 608. According to an embodiment, the electronic device 601 may include a processor 620, memory 630, an input device 650, a sound output device 655, a display device 660, an audio module 670, a sensor module 676, an interface 677, a haptic module 679, a camera module 680, a power management module 688, a battery 689, a communication module 690, a subscriber identification module(SIM) 696, or an antenna module 697. In some embodiments, at least one (e.g., the display device 660 or the camera module 680) of the components may be omitted from the electronic device 601, or one or more other components may be added in the electronic device 601. In some embodiments, some of the components may be implemented as single integrated circuitry. For example, the sensor module 676 (e.g., a fingerprint sensor, an iris sensor, or an illuminance sensor) may be implemented as embedded in the display device 660 (e.g., a display).

The processor 620 may execute, for example, software (e.g., a program 640) to control at least one other component (e.g., a hardware or software component) of the electronic device 601 coupled with the processor 620, and may perform various data processing or computation. According to one embodiment, as at least part of the data processing or computation, the processor 620 may load a command or data received from another component (e.g., the sensor module 676 or the communication module 690) in volatile memory 632, process the command or the data stored in the volatile memory 632, and store resulting data in non-volatile memory 634. According to an embodiment, the processor 620 may include a main processor 621 (e.g., a central processing unit (CPU) or an application processor (AP)), and an auxiliary processor 623 (e.g., a graphics processing unit (GPU), an image signal processor (ISP), a sensor hub processor, or a communication processor (CP)) that is operable independently from, or in conjunction with, the main processor 621. Additionally or alternatively, the auxiliary processor 623 may be adapted to consume less power than the main processor 621, or to be specific to a specified function. The auxiliary processor 623 may be implemented as separate from, or as part of the main processor 621.

The auxiliary processor 623 may control at least some of functions or states related to at least one component (e.g., the display device 660, the sensor module 676, or the communication module 690) among the components of the electronic device 601, instead of the main processor 621 while the main processor 621 is in an inactive (e.g., sleep) state, or together with the main processor 621 while the main processor 621 is in an active state (e.g., executing an application). According to an embodiment, the auxiliary processor 623 (e.g., an image signal processor or a communication processor) may be implemented as part of another component (e.g., the camera module 680 or the communication module 690) functionally related to the auxiliary processor 623.

The memory 630 may store various data used by at least one component (e.g., the processor 620 or the sensor module 676) of the electronic device 601. The various data may include, for example, software (e.g., the program 640) and input data or output data for a command related thereto. The memory 630 may include the volatile memory 632 or the non-volatile memory 634.

The program 640may be stored in the memory 630 as software, and may include, for example, an operating system (OS) 642, middleware 644, or an application 646.

The input device 650 may receive a command or data to be used by other component (e.g., the processor 620) of the electronic device 601, from the outside (e.g., a user) of the electronic device 601. The input device 650 may include, for example, a microphone, a mouse, or a keyboard.

The sound output device 655 may output sound signals to the outside of the electronic device 601. The sound output device 655 may include, for example, a speaker or a receiver. The speaker may be used for general purposes, such as playing multimedia or playing record, and the receiver may be used for an incoming calls. According to an embodiment, the receiver may be implemented as separate from, or as part of the speaker.

The display device 660 may visually provide information to the outside (e.g., a user) of the electronic device 601. The display device 660 may include, for example, a display, a hologram device, or a projector and control circuitry to control a corresponding one of the display, hologram device, and projector. According to an embodiment, the display device 660 may include touch circuitry adapted to detect a touch, or sensor circuitry (e.g., a pressure sensor) adapted to measure the intensity of force incurred by the touch.

The audio module 670 may convert a sound into an electrical signal and vice versa. According to an embodiment, the audio module 670 may obtain the sound via the input device 650, or output the sound via the sound output device 655 or a headphone of an external electronic device (e.g., an electronic device 602) directly (e.g., wiredly) or wirelessly coupled with the electronic device 601.

The sensor module 676 may detect an operational state (e.g., power or temperature) of the electronic device 601 or an environmental state (e.g., a state of a user) external to the electronic device 601, and then generate an electrical signal or data value corresponding to the detected state. According to an embodiment, the sensor module 676 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 677 may support one or more specified protocols to be used for the electronic device 601 to be coupled with the external electronic device (e.g., the electronic device 602) directly (e.g., wiredly) or wirelessly. According to an embodiment, the interface 677 may include, for example, a high definition multimedia interface (HDMI), a universal serial bus (USB) interface, a secure digital (SD) card interface, or an audio interface.

A connecting terminal 678 may include a connector via which the electronic device 601 may be physically connected with the external electronic device (e.g., the electronic device 602). According to an embodiment, the connecting terminal 678 may include, for example, a HDMI connector, a USB connector, a SD card connector, or an audio connector (e.g., a headphone connector).

The haptic module 679 may convert an electrical signal into a mechanical stimulus (e.g., a vibration or a movement) or electrical stimulus which may be recognized by a user via his tactile sensation or kinesthetic sensation. According to an embodiment, the haptic module 679 may include, for example, a motor, a piezoelectric element, or an electric stimulator.

The camera module 680 may capture a still image or moving images. According to an embodiment, the camera module 680 may include one or more lenses, image sensors, image signal processors, or flashes.

The power management module 688 may manage power supplied to the electronic device 601. According to one embodiment, the power management module 688 may be implemented as at least part of, for example, a power management integrated circuit (PMIC).

The battery 689 may supply power to at least one component of the electronic device 601. According to an embodiment, the battery 689 may include, for example, a primary cell which is not rechargeable, a secondary cell which is rechargeable, or a fuel cell.

The communication module 690 may support establishing a direct (e.g., wired) communication channel or a wireless communication channel between the electronic device 601 and the external electronic device (e.g., the electronic device 602, the electronic device 604, or the server 608) and performing communication via the established communication channel. The communication module 690 may include one or more communication processors that are operable independently from the processor 620 (e.g., the application processor (AP)) and supports a direct (e.g., wired) communication or a wireless communication. According to an embodiment, the communication module 690 may include a wireless communication module 692 (e.g., a cellular communication module, a short-range wireless communication module, or a global navigation satellite system (GNSS) communication module) or a wired communication module 694 (e.g., a local area network (LAN) communication module or a power line communication (PLC) module). A corresponding one of these communication modules may communicate with the external electronic device via the first network 698 (e.g., a short-range communication network, such as Bluetooth^{™}, wireless-fidelity (Wi-Fi) direct, or infrared data association (IrDA)) or the second network 699 (e.g., a long-range communication network, such as a cellular network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 692 may identify and authenticate the electronic device 601 in a communication network, such as the first network 698 or the second network 699, using subscriber information (e.g., international mobile subscriber identity (IMSI)) stored in the subscriber identification module 696.

The antenna module 697 may transmit or receive a signal or power to or from the outside (e.g., the external electronic device) of the electronic device 601. According to an embodiment, the antenna module 697 may include one or more antennas, from which at least one antenna appropriate for a communication scheme used in the communication network, such as the first network 698 or the second network 699, may be selected, for example, by the communication module 690 (e.g., the wireless communication module 692). The signal or the power may then be transmitted or received between the communication module 690 and the external electronic device via the selected at least one antenna.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 601 and the external electronic device 604 via the server 608 coupled with the second network 699. Each of the electronic devices 602 and 604 may be a device of a same type as, or a different type, from the electronic device 601. According to an embodiment, all or some of operations to be executed at the electronic device 601 may be executed at one or more of the external electronic devices 602, 604, or 608. For example, if the electronic device 601 should perform a function or a service automatically, or in response to a request from a user or another device, the electronic device 601, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 601. The electronic device 601 may provide the outcome, with or without further processing of the outcome, as at least part of a reply to the request. To that end, a cloud computing, distributed computing, or client-server computing technology may be used, for example.

According to the above-described embodiment, an electronic device (e.g., the electronic device 100 of FIG. 1 or the electronic device 601 of FIG. 6) may comprise a ring-shaped housing (e.g., the housing 110 of FIG. 1) having an inner surface into which a finger of a user is inserted. The electronic device may comprise a light-emitting unit (e.g., the light-emitting unit 121 of FIG. 1) configured to emit a light to the inner surface of the housing 110 through a first region of the inner surface of the housing 110 within the housing 110. The electronic device may comprise a light-receiving unit (the light-receiving units 126 and 127 of FIG. 1) configured to receive a first light, which is a portion of the light reflected from an inside of the finger of the user, through a second region of the inner surface of the housing 110 within the housing 110. The electronic device may comprise a coating layer (e.g., the first coating layer 131 or the second coating layer 132 of FIG. 1) disposed between the first region and the second region, configured to reflect a second light, which is distinct from the first light and is remaining portion of the light, to the finger of the user to transmit the second light to the light-receiving unit 126 or 127.

The electronic device 100, according to the above-described embodiment, may obtain data related to a biometric signal of the user through an optical sensor 120. The electronic device 100 may increase an amount of the light transmitted to the light-receiving units 126 and 127 by reflecting the light that may not be transmitted to the light-receiving units 126 and 127 back to the inside of the body of a user 10. As the amount of the light transmitted to the light-receiving units 126 and 127 increases, the electronic device 100 may increase accuracy of data related to the biometric signal obtained through the optical sensor 120.

According to an embodiment, the electronic device may further comprise a light-receiving unit (e.g., the additional light-receiving units 426 and 427 of FIG. 4) distinct from the light-receiving unit 126 or 127. According to an embodiment, the electronic device may further comprise a coating layer (e.g., the second coating layer 431b or the fourth coating layer 432b of FIG. 4) different from the coating layer 131, 132, 431a, or 432a, disposed between the coating layer 131, 132, 431a, or 432a and the first region.

According to an embodiment, the light-emitting unit 121 may include a first light source (e.g., the red light source 201a of FIG. 4) emitting a light of a first wavelength and a second light source (e.g., the green light source 401a of FIG. 4) emitting a light of a second wavelength shorter than the first wavelength.

According to an embodiment, the light-receiving unit 126 or 127 may be configured to receive a portion of the light of the first wavelength reflected from an inside of the finger, and the additional light-receiving unit 426 or 427 may be configured to receive a portion of the light of the second wavelength reflected from an inside of the finger.

According to the above-described embodiment, the electronic device 100 may increase accuracy of biometric data obtained through the light receiving units 126 and 127 by including coating layers 431a, 431b, 432a, and 432b. The electronic device 100 may increase accuracy of data related to a heart rate obtained through the additional light-receiving units 426 and 427 by disposing the additional light-receiving units 426 and 427 adjacently to the light-emitting unit 121.

According to an embodiment, the additional light-receiving units 426 or 427 may be disposed between the light-receiving units 126 or 127 and the light-emitting unit 121. According to the above-described embodiment, the electronic device 100 may increase the accuracy of the biometric data obtained through the light receiving units 126 and 127 by including the coating layers 431a, 431b, 432a, and 432b. The electronic device 100 may increase the accuracy of the data related to the heart rate obtained through the additional light-receiving units 426 and 427 by disposing the additional light-receiving units 426 and 427 adjacently to the light-emitting unit 121.

According to an embodiment, the electronic device may further comprise an additional light-emitting unit (e.g., the additional light-emitting unit 301 of FIG. 3) distinct from the light-emitting unit 121. The light-emitting unit 121 may include a first light source (e.g., the light source 201 of FIG. 3) emitting a light of a first wavelength.

According to an embodiment, the additional light-emitting unit 301 may include a second light source (e.g., the second light sources 301a and 301b of FIG. 3) emitting a light of a second wavelength shorter than the first wavelength. The light-receiving units 126 or 127 may be configured to receive a portion of the light of the first wavelength reflected from an inside of the finger and a portion of the light of the second wavelength reflected from an inside the finger.

According to an embodiment, the coating layers 131, 132, 431a, or 432a may be disposed between the light-emitting unit 121 and the additional light-emitting unit 301.

According to the above-described embodiment, the electronic device 100 may increase accuracy of biometric data obtained through the light-receiving units 126 and 127 by including the coating layers 131 and 132. The electronic device 100 may increase the accuracy of the data related to the heart rate obtained through the light-receiving units 126 and 127 by disposing the second light sources 301a and 301b emitting the green light adjacent to the light-receiving units 126 and 127.

According to an embodiment, the light-receiving unit 126 or 127 may be configured to obtain data related to an oxidation saturation of the user using the light of the first wavelength and obtain data related to a heart rate of the user using the light of the second wavelength.

According to the above-described embodiment, the light-receiving unit may be configured to obtain different biometric information according to a color by including a plurality of optical sensors.

According to an embodiment, an angle between the light-receiving unit 126 or 127 and the light-emitting unit 121 with respect to a center of the ring shape may be 50 to 70 degrees.

According to an embodiment, the electronic device may further include a processor (e.g., the processor 220 of FIG. 2).

According to an embodiment, the processor 220 may be configured to obtain the biometric signal of the user based on data related to the first light obtained through the light-receiving units 126 and 127.

According to an embodiment, the electronic device may further comprise a noise reduction circuit (e.g., the noise reduction circuit 240 of FIG. 2) electrically connected to the light-receiving unit 126 or 127.

According to an embodiment, the noise reduction circuit 240 may be configured to remove data related to an external light distinct from the first light, transmitted from an outside of the electronic device 100 or 601.

The electronic device 100, according to the above-described embodiment, may obtain the biometric signal of the user through the electronic device 100 including the light-emitting unit 121 and the light-receiving units 126 and 127. The electronic device 100 may guide a light that is not transmitted to the light-receiving units 126 and 127 to be reflected back to a finger 20 of the user 10 and transmitted to the light-receiving units 126 and 127 by including the coating layers 131 and 132. The electronic device 100 may improve a performance of the optical sensor 120 through the coating layers 131 and 132.

According to an embodiment, the electronic device may increase accuracy of the obtained biometric signal by removing noise related to the external light through the noise reduction circuit or a filter.

According to an embodiment, the noise reduction circuit 240 may include a high pass filter.

The electronic device, according to the above-described embodiment, may remove noise by the external light having a relatively low frequency through the noise reduction circuit, which is the high pass filter. The electronic device may obtain an accurate biometric signal by removing noise by the external light.

According to an embodiment, the light-emitting unit 121 may include one of an LED, an OLED, or a laser, and the light-receiving unit 126 or 127 may include a photodiode.

According to an embodiment, the first region and the second region of the housing 110 may include a transparent portion.

The electronic device, according to the above-described embodiment, may penetrate a light emitted from the light-emitting unit or a light transmitted to the light-receiving unit through the transparent portion formed in the inner surface of the housing.

According to an embodiment, the electronic device may further comprise a flexible printed circuit board (e.g., the FPCB 210 of FIG. 2) on which the light-emitting unit 121 and the light-receiving unit 126 or 127 are disposed, bent along an interior of the housing 110.

According to the above-described embodiment, the flexible printed circuit board 210 may electrically connect electronic components disposed inside the electronic device.

According to an embodiment, the light-receiving unit 126 or 127 may include a plurality of optical sensors, and a distance between a first optical sensor, which is one of the plurality of light sensors, and the light-emitting unit 121 may be the same as a distance between a second optical sensor, which is another of the plurality of light sensors, and the light-emitting unit 121.

According to an embodiment, the coating layer 131, 132, 431a or 432a may include a first coating disposed between the first optical sensor and the light-emitting unit 121 and a second coating disposed between the second optical sensor and the light-emitting unit 121.

According to an embodiment, the electronic device (e.g., the electronic device 100 of FIG. 1 or the electronic device 601 of FIG. 6) may comprise a ring-shaped housing (e.g., the housing 110 of FIG. 1) having an inner surface into which a finger of a user is inserted. The electronic device may comprise a cover, forming at least a portion of the inner surface of the housing 110, including a transparent portion. According to an embodiment, the electronic device 100 may comprise a light-emitting unit (e.g., the light-emitting unit 121 of FIG. 1) configured to receive a first light reflected from an inside of the finger of the user through a second region of the cover within the housing 110. The electronic device may include a light-receiving unit (e.g., the light-receiving units 126 and 127 of FIG. 1) configured to receive the first light reflected from the inside the finger of the user through the second region of the cover in the housing 110. According to an embodiment, the electronic device may comprise an additional light-receiving unit (e.g., the additional light-receiving units 426 and 427 of FIG. 4) disposed between the light-receiving unit 126 or 127 and the light-emitting unit 121, configured to receive a second light, which is distinct from the first light and is reflected from an inside of the finger of the user, through a third region of the cover within the housing 110. The electronic device may comprise a coating layer (e.g., the coating layer 131 or 132 of FIG. 1 or the coating layers 431a and 432a of FIG. 4) disposed on the cover and between the first region and the second region, configured to reflect a third light, which is distinct from the first light and the second light and is reflected from an inside of the finger of the user, to the finger of the user to transmit the third light to the light-receiving unit 126 or 127. According to an embodiment, the electronic device may further comprise another coating layer 431b or 432b, different from the coating layer 131, 132, 431a or 432a, disposed between the coating layer 131, 132, 431a, or 432a and the first region.

According to an embodiment, the light-emitting unit 121 may include a first light source emitting the first light of a first wavelength and a second light source emitting the second light of a second wavelength shorter than the first wavelength.

According to an embodiment, the light-receiving unit 126 or 127 may be configured to receive a portion of the first light of the first wavelength, reflected from an inside of the finger.

According to an embodiment, the light-receiving unit 426 or 427 may be configured to receive a portion of the second light of the second wavelength, reflected from an inside of the finger.

According to an embodiment, an angle between the light-receiving unit 126 or 127 and the light-emitting unit 121 with respect to a center of the ring shape may be 50 to 70 degrees.

According to an embodiment, the electronic device may further include a processor 220.

According to an embodiment, the processor 220 may be configured to obtain data related to an oxidation saturation of the user based on data related to the first light obtained through the light-receiving unit 126 or 127.

According to an embodiment, it may be configured to obtain data related to a heart rate of the user based on data related to the second light obtained through the additional light-receiving unit 426 or 427.

According to an embodiment, the light-emitting unit 121 may include one of an LED, an OLED, or a laser. The light-receiving unit 126 or 127 and the light-receiving unit 426 or 427 may include a photodiode.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 640) including one or more instructions that are stored in a storage medium (e.g., internal memory 636 or external memory 638) that is readable by a machine (e.g., the electronic device 601). For example, a processor (e.g., the processor 620) of the machine (e.g., the electronic device 601) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device (100; 601) comprising:
a ring-shaped housing (110) having an inner surface into which a finger of a user is inserted;
a light-emitting unit (121) configured to emit a light to the inner surface of the housing (110) through a first region of the inner surface of the housing (110) within the housing (110);
a light-receiving unit (126; 127) configured to receive a first light, which is a portion of the light reflected from an inside of the finger of the user, through a second region of the inner surface of the housing (110) within the housing (110); and
a coating layer (131; 132; 431a; 432a), disposed between the first region and the second region, configured to reflect a second light, which is distinct from the first light and is remaining portion of the light, to the finger of the user to transmit the second light to the light-receiving unit (126; 127).

2. The electronic device (100; 601) of claim 1, comprising:
an additional light-receiving unit (426; 427) distinct from the light-receiving unit (126; 127); and
another coating layer (431b; 432b), different from the coating layer (131; 132; 431a; 432a), disposed between the coating layer (131; 132; 431a; 432a) and the first region,
wherein the light-emitting unit (121) includes a first light source emitting a light of a first wavelength and a second light source emitting a light of a second wavelength shorter than the first wavelength,
wherein the light-receiving unit (126; 127) is configured to receive a portion of the light of the first wavelength reflected from an inside of the finger,
wherein the additional light-receiving unit (426; 427) is configured to receive a portion of the light of the second wavelength reflected from an inside of the finger.

3. The electronic device (100; 601) of claim 1 or claim 2,
wherein the additional light-receiving unit (426; 427) is disposed between the light-receiving unit (126; 127) and the light-emitting unit (121).

4. The electronic device (100; 601) of any one of claims 1 to 3, further comprising:
an additional light-emitting unit (301) distinct from the light-emitting unit (121),
wherein the light-emitting unit (121) includes a first light source emitting a light of a first wavelength,
wherein the additional light-emitting unit (301) includes a second light source emitting a light of a second wavelength shorter than the first wavelength,
wherein the light-receiving unit (126; 127) is configured to receive a portion of the light of the first wavelength reflected from an inside of the finger and a portion of the light of the second wavelength reflected from an inside the finger.

5. The electronic device (100; 601) of any one of claims 1 to 4,
wherein the coating layer (131; 132; 431a; 432a) is disposed between the light-emitting unit (121) and the additional light-emitting unit (301).

6. The electronic device (100; 601) of any one of claims 1 to 5,
wherein the light-receiving unit (126; 127) is configured to obtain data related to an oxidation saturation of the user using the light of the first wavelength and obtain data related to a heart rate of the user using the light of the second wavelength.

7. The electronic device (100; 601) of any one of claims 1 to 6,
wherein an angle between the light-receiving unit (126; 127) and the light-emitting unit (121) with respect to a center of the ring shape is 50 to 70 degrees.

8. The electronic device (100; 601) of any one of claims 1 to 7, further comprising a processor (220),
wherein the processor (220) obtains a biometric signal of the user based on data related to the first light obtained through the light-receiving unit (126; 127).

9. The electronic device (100; 601) of any one of claims 1 to 8, further comprising a noise reduction circuit (240) electrically connected to the light-receiving unit (126; 127),
wherein the noise reduction circuit (240) is configured to remove data related to an external light, distinct from the first light, transmitted from an outside of The electronic device (100; 601).

10. The electronic device (100; 601) of any one of claims 1 to 9,
wherein the noise reduction circuit (240) includes a high pass filter.

11. The electronic device (100; 601) of any one of claims 1 to 10,
wherein the light-emitting unit (121) includes at least one of an LED, an OLED, or a laser,
wherein the light-receiving unit (126; 127) includes a photo diode.

12. The electronic device (100; 601) of any one of claims 1 to 11,
wherein the first region and the second region of the housing (110) includes a transparent portion.

13. The electronic device (100; 601) of any one of claims 1 to 12, further comprising a flexible printed circuit board (210) on which the light-emitting unit (121) and the light-receiving unit (126; 127) are disposed, bent along an interior of the housing (110).

14. The electronic device (100; 601) of any one of claims 1 to 13,
wherein the light-receiving unit (126; 127) includes a plurality of light sensors,
wherein a distance between a first light sensor, which is one of the plurality of light sensors, and the light-emitting unit (121) is the same as a distance between a second light sensor, which is another of the plurality of light sensors, and the light-emitting unit (121).

15. The electronic device (100; 601) of any one of claims 1 to 14,
wherein the coating layer (131; 132; 431a; 432a) includes a first coating disposed between the first light sensor and the light-emitting unit (121) and a second coating disposed between the second light sensor and the light-emitting unit (121).
